# EUROPÄISCHE PATENTSCHRIFT

(11) **EP 1 843 994 B1**
(45) Veröffentlichungstag und Bekanntmachung des Hinweises auf die Patenterteilung: **19.03.2014**
(21) Anmeldenummer: 05784695.8
(22) Anmeldetag: 13.09.2005
(51) Int. Cl.: C07C 2/46

(54) **VERFAHREN ZUR HERSTELLUNG VON TRIMETHYLCYCLODODECATRIEN UNTER VERWENDUNG EINES TITAN-KATALYSATORS**
METHOD FOR PRODUCING TRIMETHYLCYCLODODECATRIENE WITH THE USE OF A TITANIUM CATALYST
PROCEDE DE PRODUCTION DE TRIMETHYLCYCLODODECATRIENE PAR UTILISATION D'UN CATALYSEUR A BASE DE TITANE

(30) Priorität: 11.11.2004 DE 102004054477
(43) Veröffentlichungstag der Anmeldung: 17.10.2007
(73) Patentinhaber: Evonik Degussa GmbH, 45128 Essen (DE)
(72) Erfinder: KUPPERT, Dirk, 45657 Recklinghausen (DE); HERWIG, Jürgen, 46569 Hünxe (DE); WILCZOK, Norbert, 45481 Mülheim (DE); VÖLKEL, Anita, 45701 Herten (DE); THIERY, Manfred, 48653 Coesfeld (DE)
(86) Internationale Anmeldenummer: PCT/EP2005/054541
(87) Internationale Veröffentlichungsnummer: WO 2006/051011

(56) Entgegenhaltungen:
- DE-A1- 2 951 508
- DE-B- 1 140 569
- FR-A- 1 393 071
- GB-A- 928 812
- US-A- 3 499 049
- US-A- 3 723 478
- US-A- 4 020 118
- N. PLATZER, S. INOUE: "Terpene Product Derivatives from Isoprene Oligomerisation" IND. ENG. CHEM. PROD. RES. DEV., Bd. 18, Nr. 4, 1979, Seiten 254-258, XP002362184 in der Anmeldung erwähnt
- DATABASE CA [Online] CHEMICAL ABSTRACTS SERVICE, COLUMBUS, OHIO, US; TAKAHASHI, HIROSHI ET AL: "Cyclododecatriene" XP002362185 gefunden im STN Database accession no. 1975:139520 & JP 49 042497 B (AGENCY OF INDUSTRIAL SCIENCES AND TECHNOLOGY) 15. November 1974 (1974-11-15)

## Beschreibung

Die vorliegende Erfindung betrifft ein kontinuierliches und diskontinuierliches Verfahren zur Herstellung cyclischer Isopren-Trimere, d. h. 1,5,9- und 1,6,9-Trimethylcyclododecatrien-(1,5,9) (TMCDT) unter Verwendung eines Katalysatorsystems enthaltend Titan. Des Weiteren umfasst die vorliegende Erfindung das zuvor genannte Verfahren, bei dem zusätzlich 1,5-und/oder 1,6 Dimethylcyclooctadien (DMCOD) aus der Reaktionsmischung isoliert werden kann.

Eine große Zahl an Patenten und Publikationen belassen sich mit Verfahren bzw. Versuchen zur Herstellung von cyclischen Di- und Trimeren konjugierter Diene, insbesondere Butadien.

Im Gegensatz zum Butadien ist die Di- und Trimerisierung von Isopren, z. B. die Umwandlung zu Dimethylvinylcyclohexen und Dimethylcyclooctadien relativ wenig erforscht. Die Synthese von Trimethylcyclododecatrien aus Isopren wurde bislang wenig untersucht. Es wurde berichtet über den Einsatz von Katalysatorsystemen enthaltend Titan oder auch Nickel.

Die Bildung von Trimethylcyclododecatrien in Gegenwart eines Titan Katalysators wurde zum Beispiel in der JP 2003064001 beschrieben. Dabei wurde die Reaktion unter Verwendung eines Katalysatorsystems aus Titantetrachlorid, 4,4'-Dichlorbenzophenon, Dimethylsulfoxid und Diethylaluminiumsesquichlorid bei einer Temperatur von 40°C durchgeführt. Nach beendeter Reaktion wurde die Mischung mit MeONa/MeOH versetzt und mit wässrigem Trinatriumcitrat gewaschen um das Titan und Aluminium aus der organischen Reaktionsmischung zu entfernen. Die Gegenwart des hochsiedenden DMSO als Additiv ist nachteilig für einen industriellen Prozess, da es aus der Reaktionsmischung wieder entfernt werden muss.
Die DE 2833367 beschreibt die Herstellung von TMCDT unter Verwendung eines Katalysators, hergestellt aus einer dreiwertigen Titanverbindung, einer Organoaluminiumverbindung, einer Sauerstoff-enthaltenden Verbindung wie z.B. Carbonyl- oder Etherverbindungen und einer weiteren Komponente enthaltend Schwefel oder eine Nitrilfunktion. Der Einsatz einer dreiwertigen Titanverbindung ist nachteilig für industrielle Prozesse, da diese Verbindungen sehr feuchtigkeitsempfindlich, damit schwer zu handhaben und leicht deaktivierbar sind. Weiterhin wurde in allen Beispielen über die Bildung von polymeren Nebenprodukten durch Polymerisation von Isopren berichtet, auch wenn die Menge nicht angegeben wurde. Die Bildung solcher polymeren Nebenprodukte sollte so gering wie möglich gehalten werden, da dadurch die Ausbeute an TMCDT reduziert wird.

FR 1393071 beschreibt die Bildung von TMCDT mit Titan und Aluminium als Katalysatorsystem. Als Titankatalysator wurde Ti(OR)₄ verwendet, wobei R ein aliphatischer C₃ - C₄-Alkylrest ist, als Aluminium Katalysator wurde AIR'X₂ oder AIR'₂X verwendet, wobei R' ein geradkettiger oder verzweigter C₁ - C₁₈ Alkylrest oder ein C₁ - C₆-Cycloalkyl oder ein C₁ - C₁₀ Aralkylrest und X gleich Cl oder Br ist. Die in der FR1393071 beschriebene Reaktion benötigt sehr lange Reaktionszeiten von 18h und ist daher für industrielle Anwendungen nicht geeignet. Ferner werden in der französischen Patentschrift keine Ausbeuten angegeben.

In der DE 1 050 333 wird ein Titan basierter Katalysator ohne Zusatz von Additiven wie zum Beispiel Schwefel oder Stickstoff-enthaltende Verbindungen benutzt. Reaktionszeiten von 12h bei Ausbeuten von 45 - 50% TMCDT wurden erreicht. Somit benötigt auch dieses Verfahren zu lange Reaktionszeiten.

In Ind. Eng. Chem. Res. Dev., Vol. 18, No. 4, (1979) Seite 254 wird die Synthese von TMCDT aus Isopren unter Verwendung eines Titankatalysators lediglich erwähnt. Eindeutige experimentelle Details und Ausbeuten sind nicht beschrieben. Die Bildung von C₁₀-Dimeren von Isopren wie zum Beispiel 2,4-Dimethyl-4-vinylcyclohexen-1 und des linearen 2,6-Dimethyloctatrien-1,3,6 in Abhängigkeit von der Zugabe von cyclischen Ethern zur Reaktionsmischung wird ebenfalls beschrieben. Es wurde beobachtet, dass die Ausbeute an Dimer steigt, wenn die Lewisbasizität der Sauerstoff-tragenden Donoren steigt. In besagter Publikation wird ebenfalls die Nickel katalysierte Reaktion von Isopren zu TMCDT und DMCOD in Gegenwart von Phosphinen oder Phosphiten beschrieben ohne dass experimentelle Details genannt werden.

In JP 7456950, JP 7698242 und JP 7456951 werden Nickel katalysierte Systeme offenbart. Titan katalysierte Umsetzung von Isopren zu TMCDT wurde nicht erwähnt.

Die US 3,804,913 und die US 3,429,940 offenbaren lediglich den Einsatz von Chromkatalysatoren bei der Umwandlung von Isopren in TMCDT. Die Ausbeute dieser Reaktionen lag bei 32 bis 50%. Chrom basierte Systeme weisen den Nachteil hoher Toxizität auf.

In Bull.-Chem. Soc. Jpn. 1978, 1158 wird ein Nickel katalysiertes System beschrieben, wobei die Ausbeuten lediglich 7,2 % betrugen.

Die DE 1140569 offenbart die Bildung von Dimeren und Trimeren von 1,3-Diolefinen mittels Nickel- oder Cobaltkatalysatorsystemen. Die Katalysatorsysteme enthalten zudem organometallische Verbindungen sowie Verbindungen mit Elektronendonoreigenschaften. Gemäß DE 1140569 kann das Verhältnis von Dimer zu Trimer durch die Wahl geeigneter Reaktionsparameter beeinflusst werden. Im Fall von Isopren als 1,3-Diolefin wurde in der DE 1140569 fast ausschließlich die Bildung von 2,6-Dimethylcyclooctadien-(1,5) und 2,5-Dimethylcyclooctadien-(1,5) beobachtet (siehe Beispiel 52). In Beispiel 64 betrug die Ausbeute an TMCDT ebenfalls nur 18,1%. Das Verfahren gemäß DE 1140569 benötigt den Einsatz von absolutierten Lösungsmitteln, was mit erheblichem technischen Aufwand und somit ökonomischen Nachteilen verbunden ist.

Bei der großtechnischen Trimerisierung von Butadien zu Cyclododecatrien (CDT) werden homogene Katalysatoren benutzt, wobei die Reaktion in einem kontinuierlichen Prozess in einem oder mehreren gerührten Kesseln durchgeführt wird. Dabei werden Teile der Reaktionsmischung kontinuierlich aus der Reaktionsmischung entnommen. Während der Aufarbeitung wird unreagiertes Ausgangsmaterial zurückgewonnen und dem Kreislauf zusammen mit frischem Butadien erneut zugeführt. Teile des Katalysators werden bei der Entnahme ebenfalls dem Reaktionsgemisch entzogen. Dadurch sinkt die Konzentration des Katalysators in der Reaktionsmischung und muss durch frischen Katalysator ersetzt werden um die Katalysatorkonzentration konstant zu halten.

Vor der Aufarbeitung des aus dem Reaktor entnommenen Materials muss der entnommene Katalysator zerstört werden. Eine Vielzahl polarer Lösungsmittel werden hierzu eingesetzt. Neben Wasser, benutzt Ube Industries z. B. Ammoniumhydroxid Lösungen (JP 05-070377, JP 06-25438). Verschiedene Alkohole können ebenfalls benutzt werden (JP 07-625439, JP 07-625396). Insbesondere Methanol (JP 07-442496) und Methanol/HCl (DE 19 42 729) kommen bevorzugt zum Einsatz.

Die Zersetzung des Katalysators kann auch mittels Aceton (JP 04-301345) oder mittels einer Suspension von Calziumoxid in Wasser (NL 6 603 264) durchgeführt werden. Ube Industries berichtete zudem, dass die Ausbeute an CDT sinkt, wenn Wasser zur Zersetzung des Katalysators verendet wird.

Ausgehend vom zuvor genannten Stand der Technik war es daher die Aufgabe der vorliegenden Erfindung ein Verfahren zur Herstellung vom Trimethylcyclododecatrien (TMCDT) mit hohen Ausbeuten und niedriger Menge an polymeren Nebenprodukten bereitzustellen. Eine weitere Aufgabe bestand darin ein Verfahren bereitzustellen, bei dem die Menge an C₁₀-Dimeren, z. B. Dimethylvinylcyclohexen im Falle eines Titan katalysierten Systems niedrig ist. Eine weitere Aufgabe bestand schließlich darin, ein Verfahren bereitzustellen, dass es bei katalysierten Systemen, erlaubt neben hohen Mengen an TMCDT auch Dimethylcyclooctadien zu isolieren.

Diese und weitere nicht explizit genannte Aufgaben sowie deren Lösung werden durch die nachfolgende Beschreibung sowie die Ansprüche näher erläutert.

Es wurde nun überraschend gefunden, dass Übergangsmetallkomplexe des Titans Isopren mit hoher Selektivität zu TMCDT trimerisieren können. Um diese hohe Selektivität erreichen zu können, ist es notwendig, dass Ammoniak und ein geeignetes Lösungsmittelsystem verwendet werden. Ferner wurde gefunden, dass die Reaktionstemperatur im Falle von Titan katalysierten Systemen unterhalb von 80°C liegen sollte.

Gegenstand der Erfindung ist daher ein Verfahren zur Herstellung von TMCDT aus Isopren in Gegenwart eines Katalysatorsystems, welches durch die Ansprüche 1 bis 16 definiert und durch die nachfolgende Beschreibung näher spezifiziert ist. Gegenstand der vorliegenden Erfindung ist insbesondere ein kontinuierliches und/oder diskontinuierliches Verfahren zur Herstellung von Trimethylcyclododecatrien, durch Umsetzung von Isopren in Gegenwart eines Lösungsmittels, zumindest eines Katalysatorsystems enthaltend Nickel und/oder Titan sowie zumindest einer Organometallverbindung zu einem roh-Trimethylcyclododecatrien,
dadurch gekennzeichnet,
dass
- Ammoniak zugegeben wird,
- das Lösungsmittel vor der Zugabe der Katalysatorkomponente 10 - 1000 ppm einer polaren Komponente der allgemeinen Formel HO-R, wobei R ausgewählt ist aus der Gruppe, die aus verzweigtem und unverzweigte C₁ - C₁₈-Alkyl, C₁ - C₁₈-Cycloalkyl, C₁ - C₁₈-Aryl, C₁ - C₁₈-Aralkyl und H besteht, enthält, und
- dass die Reaktionstemperatur im Falle eines Titan-enthaltenden Katalysatorsystems kleiner gleich 80°C beträgt.

Ebenfalls Gegenstand der vorliegenden Erfindung ist ein Verfahren bei dem neben TMCDT auch DMCOD aus der Reaktionsmischung isoliert werden kann.

Stoffgemische erhältlich nach dem erfindungsgemäßen Verfahren sind dadurch gekennzeichnet, dass das Verhältnis von Trimethylcyclododecatrien zu linearen C15-Oligomeren des Isoprens - ermittelt mittels Gaschromatographie (DB1-Säule) - größer gleich 10:1, bevorzugt größer als 15:1, besonders bevorzugt größer als 20 zu 1 ist. Weiterhin bevorzugt ist das Verhältnis von Dimethylcyclododecatrien zu linearen C15-Oligomeren kleiner als 60:1 und besonders bevorzugt kleiner als 50:1. Diese Stoffgemische können im Falle von Titan als Katalysator bevorzugt cis,trans,trans-TMCDT enthalten.

Wie im Nachfolgenden bestätigt werden wird, ermöglicht das erfindungsgemäße Verfahren die Herstellung von Trimeren des Isoprens, insbesondere 1,5,9- und 1,6,9-Trimethylcyclododecatrien-(1,5,9) (TMCDT) mit hoher Selektivität und hohen Ausbeuten, wobei sehr kurze Reaktionszeiten benötigt werden. Weiterhin konnte durch das erfindungsgemäße Verfahren die Menge an polymeren Nebenprodukten reduziert werden. Schließlich führt das erfindungsgemäße Verfahren zu Produktgemischen, wobei das Verhältnis von TMCDT zu linearen C₁₅-Oligomeren optimiert ist, d. h. ≥10:1, bevorzugt ≥ 15:1, besonders bevorzugt ≥ 20: ist.

Ausgangsmaterialien für die Katalysatorsysteme des erfindungsgemäßen Verfahrens sind bevorzugt kommerziell erhältliche Titan (IV)-Verbindungen. Besonders bevorzugt ist Titantetrachlorid.

Die Reaktion wird durchgeführt bei Katalysatorkonzentrationen von 0.01 bis 40 mmol/l, bevorzugt 0.05 bis 10 mmol/l basierend auf Titan.

Die organometallischen Verbindungen beinhalten zumindest ein Element der 1. bis 3. Hauptgruppe des Periodensystems der Elemente, bevorzugt Aluminium. Besonders bevorzugt sind Ethoxydiethylaluminium und Ethylaluminiumsesquichlorid.

Im Falle von Titan katalysierten Reaktionen beträgt das molare Verhältnis Titan zu Organometallverbindung 1:10 bis 1:60, bevorzugt 1:10 bis 1:40. Die Reaktionstemperatur beträgt kleiner gleich 80°C, bevorzugt 20 bis 80°C, besonders bevorzugt 30 - 70 °C.

Ammoniak kann als Reinstoff oder in Form von wässrigen Lösungen zugesetzt werden. Das Verhältnis von Ammoniak zur Titanverbindung wird bevorzugt so gewählt, dass das molare Verhältnis von Titan zu Ammoniak im Bereich von 1:3 bis 1:60, besonders bevorzugt 1:5 bis 1:20 liegt.

Die im erfindungsgemäßen Verfahren benutzten Lösungsmittel umfassen gesättigte und ungesättigte Lösungsmittel, unpolare aprotische Lösungsmittel, aliphatische und aromatische Kohlenwasserstoffe sowie Mischungen davon. Nicht limitierende Beispiele dafür sind Toluol, Benzol, Xylol, Hexane, Oktane, Cyclohexane, Cyclooctane, Cyclooctadiene und Mischungen davon. Das Lösungsmittel hat eine Konzentration von 10 bis 95 Gewichtsprozent in der Mischung am Ende der Reaktion oder während der Reaktion, wenn die Reaktion kontinuierlich durchgeführt wird. Das Lösungsmittel muss eine geringe Menge einer polaren Komponente der allgemeinen Formel HO-R enthalten, wobei R ausgewählt ist aus der Gruppe, die aus verzweigtem und unverzweigtem C₁ - C₁₈-Alkyl, C₁ - C₁₈-Cycloalkyl, C₁ - C₁₈-Aryl, C₁ - C₁₈-Aralkyl und H besteht, wobei die Kohlenstoffatome der Alkyl-, Cycloalkyl-, Aryl- und Aralkylreste durch ein Heteroatom, insbesondere O, N oder S ersetzt sein können bzw. wobei die Kohlenstoffatome Hydroxylgruppen, Aminogruppen und/oder Halogenatome tragen können. Besonders bevorzugt ist R ausgewählt aus der Gruppe, die aus Methyl, Ethyl, Propyl, Isopropyl, Butyl, Isobutyl, Tertbutyl und H besteht. Bevorzugt sind im Lösungsmittel 10 bis 500 ppm und 10 bis 250 ppm der polaren Komponente enthalten.

Das erfindungsgemäße Verfahren kann in Druckbereichen von 1 - 20 bar, bevorzugt 1 bis 10 bar betrieben werden. Der Betriebsdruck kann durch die Reaktionstemperatur und/oder aufpressen von Inertgasen, bevorzugt Stickstoff, eingestellt werden.

Das erfindungsgemäße Verfahren kann kontinuierlich oder diskontinuierlich betrieben werden, wobei die Zugabe der einzelnen Komponenten bevorzugt in der folgenden Reihenfolge erfolgt:
- zunächst das Lösungsmittel inkl. polarer Komponente, danach zumindest eine Organometallverbindung, danach zumindest eine Titan- enthaltende Verbindung, danach Ammoniak und danach Isopren.
- zunächst das Lösungsmittel inkl. polarer Komponente, danach zumindest eine Titan- enthaltende Verbindung, danach zumindest eine Organometallverbindung, danach Ammoniak und danach Isopren.
- zunächst das Lösungsmittel inkl. polarer Komponente, danach Ammoniak; danach zumindest eine Titan- enthaltende Verbindung, danach zumindest eine Organometallverbindung, und danach Isopren.

Die Zugabe der einzelnen Komponenten kann mit und ohne zeitliche Verzögerung erfolgen. Es ist möglich, alle Komponenten in kurzer Zeit zuzugeben und anschließend nach zu rühren, bis die Reaktion beendet ist. Es ist jedoch auch möglich, die einzelnen Komponenten über einen längeren Zeitraum zuzugeben wodurch eine kürzere Nachrührzeit benötigt wird. Kombinationen der beiden Ausführungsformen sind ebenfalls möglich. Das Nachrühren erfolgt bevorzugt bei der gleichen Temperatur bei der die einzelnen Komponenten zugegeben wurden. Bevorzugt wird das Isopren so zugegeben, dass die Temperatur konstant auf einen bestimmten Wert gehalten wird. Die Reaktion wird bevorzugt fortgeführt bis > 90%, besonders bevorzugt > 95% des Isoprens - nach gaschromatographischer Analyse - umgesetzt sind.

Das erhaltene TMCDT bzw. die erhaltenen Stoffgemische enthaltend TMCDT werden bevorzugt zur Herstellung von Duftstoffen und/oder Parfümen verwendet.

Die nachfolgenden Beispiele dienen zur näheren Erläuterung der Erfindung.

### Beispiele:

### Beispiel 1 (Vergleichsbeispiel)

Ein 1.5-Liter Glasautoklav, mit Stickstoffatmosphäre, wurde mit 0.5 Liter Benzol mit einem Wassergehalt von 15 ppm befüllt. Danach wurden 42.5 ml Ethylaluminium-sesquichlorid (20%ige Lösung in Benzol) und 13.2 ml TiCl₄ (5%ige Lösung in Benzol) zugefügt. Der Autoklav wurde verschlossen und die Reaktionsmischung auf 40°C erwärmt. Bei dieser Temperatur wurden 343 g Isopren binnen 4 Stunden zugegeben. Während der Zugabe von Isopren wurde die Temperatur im Autoklaven konstant bei 40°C gehalten. Nach beendeter Zugabe wurde die Reaktionsmischung für 1 Stunde bei 40°C weiter gerührt und schließlich der Katalysator durch Zugabe von 25 %iger wässriger Natronlauge zersetzt. Die organische Reaktionsmischung enthielt 48.81 % TMCDT, sowie 3.48 % cyclische und lineare C₁₀-Dimere und 4.85% lineare Trimere und 29.4% polymere Bestandteile.

### Beispiel 2 (erfindungsgemäß)

Ein 1.5-Liter Glasautoklav, mit Stickstoffatmosphäre, wurde mit 0.5 Liter Benzol mit einem Wassergehalt von 15 ppm befüllt. Danach wurden 84.9 ml Ethylaluminium-sesquichlorid (20%ige Lösung in Benzol) und 13.2 ml TiCl₄ (5%ige Lösung in Benzol) zugefügt. Der Autoklav wurde verschlossen und bei Raumtemperatur unter Rühren 201 mg Ammoniakgas durch ein Septum zugegeben. Die Reaktionsmischung wurde auf 40°C erwärmt. Bei dieser Temperatur wurden 170.5 g Isopren binnen 4 Stunden zugegeben. Während der Zugabe von Isopren wurde die Temperatur im Autoklaven konstant bei 40°C gehalten. Nach beendeter Zugabe wird die Reaktionsmischung für 1 Stunde bei 40°C weiter gerührt und schließlich der Katalysator durch Zugabe von 25 %iger wässriger Natronlauge zersetzt. Die organische Reaktionsmischung enthielt 60.27 % TMCDT, sowie 14.07 % cyclische und lineare C₁₀-Dimere und 4.14% lineare Trimere und 13.35% polymere Bestandteile.

### Beispiel 3 (erfindungsgemäß)

Ein 10-Liter Autoklav aus rostfreiem Stahl, mit Stickstoffatmosphäre, wurde mit 4 Liter Benzol mit einem Wassergehalt von 15 ppm befüllt. Danach wurden 76.8 ml Ethylaluminium-sesquichlorid (20%ige Lösung in Benzol) und 16.3 ml TiCl₄ (5%ige Lösung in Benzol) zugefügt. Der Autoklav wurde verschlossen und bei Raumtemperatur unter Rühren 296 mg Ammoniakgas durch ein Septum zugegeben. Die Reaktionsmischung wurde auf 40°C erwärmt. Bei dieser Temperatur wurden 2716g Isopren zugegeben mit einer Zuflussrate von 7g/min zugegeben. Während der Zugabe von Isopren wurde die Temperatur im Autoklaven konstant bei 40°C gehalten. Nach beendeter Zugabe wird die Reaktionsmischung für 2 Stunde bei 40 °C weiter gerührt und schließlich der Katalysator durch Zugabe von 25 %iger wässriger Natronlauge zersetzt. Die organische Reaktionsmischung enthielt 53.12% TMCDT, sowie 9.85% cyclische und lineare C₁₀-Dimere und 2.29% lineare Trimere.

### Beispiel 4 (erfindungsgemäß)

Ein 10-Liter Autoklav aus rostfreiem Stahl, mit Stickstoffatmosphäre, wurde mit 4 Liter Benzol mit einem Wassergehalt von 50 ppm befüllt. Danach wurden 76.8 ml Ethylaluminium-sesquichlorid (20%ige Lösung in Benzol) und 16.3 ml TiCl₄ (5%ige Lösung in Benzol) zugefügt. Der Autoklav wurde verschlossen und bei Raumtemperatur unter Rühren 296 mg Ammoniakgas durch ein Septum zugegeben. Die Reaktionsmischung wurde auf 40°C erwärmt. Bei dieser Temperatur wurden 2716g Isopren zugegeben mit einer Zuflussrate von 7g/min zugegeben. Während der Zugabe von Isopren wurde die Temperatur im Autoklaven konstant bei 40°C gehalten. Nach beendeter Zugabe wird die Reaktionsmischung für 2 Stunde bei 40 °C weiter gerührt und schließlich der Katalysator durch Zugabe von 25 %iger wässriger Natronlauge zersetzt. Die organische Reaktionsmischung enthielt 71.91% TMCDT, sowie 4.2% cyclische und lineare C₁₀-Dimere und 1,82% lineare Trimere.

### Beispiel 5 (erfindungsgemäß)

Ein 10-Liter Autoklav aus rostfreiem Stahl, mit Stickstoffatmosphäre, wurde mit 4 Liter Benzol mit einem Wassergehalt von 110 ppm befüllt. Danach wurden 76.8 ml Ethylaluminium-sesquichlorid (20%ige Lösung in Benzol) und 16.3 ml TiCl₄ (5%ige Lösung in Benzol) zugefügt. Der Autoklav wurde verschlossen und bei Raumtemperatur unter Rühren 296 mg Ammoniakgas durch ein Septum zugegeben. Die Reaktionsmischung wurde auf 40°C erwärmt. Bei dieser Temperatur wurden 2716g Isopren zugegeben mit einer Zuflussrate von 7g/min zugegeben. Während der Zugabe von Isopren wurde die Temperatur im Autoklaven konstant bei 40°C gehalten. Nach beendeter Zugabe wird die Reaktionsmischung für 2 Stunde bei 40 °C weiter gerührt und schließlich der Katalysator durch Zugabe von 25 %iger wässriger Natronlauge zersetzt. Die organische Reaktionsmischung enthielt 66.39% TMCDT, sowie 5,31 % cyclische und lineare C₁₀-Dimere und 0,95% lineare Trimere.

### Beispiel 6 (Vergleichsbeispiel)

Ein 1.5-Liter Glasautoklav, mit Stickstoffatmosphäre, wurde mit 150ml Toluol mit einem Wassergehalt von 15 ppm befüllt. Danach wurden 16.7 ml Diethylaluminiumethoxide (50%ige Lösung in COD) und 4.06g Nickelacetylacetonat sowie 5.06g 1,1,1-Trismethylolpropanphosphit zugefügt. Der Autoklav wurde verschlossen und die Reaktionsmischung auf 90°C erwärmt. Bei dieser Temperatur wurden 400 g Isopren binnen 4 Stunden zugegeben. Während der Zugabe von Isopren wurde die Temperatur im Autoklaven konstant bei 90°C gehalten. Nach beendeter Zugabe wurde die Reaktionsmischung für 20 Stunden bei 90°C weiter gerührt und schließlich der Katalysator durch Zugabe von 25 %iger wässriger Natronlauge zersetzt. Die organische Reaktionsmischung enthielt 12.16% DMCOD und 63.06% TMCDT, sowie 3.78 % cyclische und lineare C₁₀-Dimere und 6.39% lineare Trimere.

## Patentansprüche

1. Ein kontinuierliches und/oder diskontinuierliches Verfahren zur Herstellung von Trimethylcyclododecatrien, durch Umsetzung von Isopren in Gegenwart eines Lösungsmittels, zumindest eines Katalysatorsystems sowie zumindest einer Organometallverbindung zu einem roh-Trimethylcyclododecatrien,
**dadurch gekennzeichnet,**
**dass**
- das Katalysatorsystem Titan enthält
- Ammoniak zum Katalysatorsystem zugegeben wird,
- das Lösungsmittel vor der Zugabe der Katalysatorkomponente 10 - 1000 ppm einer polaren Komponente der allgemeinen Formel HO-R, wobei R ausgewählt ist aus der Gruppe, die aus verzweigtem und unverzweigtem C₁ - C₁₈-Alkyl, C₁ - C₁₈-Cycloalkyl, C₁ - C₁₈-Aryl, C₁ - C₁₈-Aralkyl und H besteht, enthält und
- **dass** die Reaktionstemperatur kleiner gleich 80°C beträgt.

2. Verfahren nach Anspruch 1,
**dadurch gekennzeichnet,**
**dass** das Katalysatorsystem Titantetrachlorid enthält.

3. Verfahren nach einem der Ansprüche 1 bis 2,
**dadurch gekennzeichnet,**
**dass** die Reaktion bei 30 bis 70°C durchgeführt wird.

4. Verfahren nach einem der Ansprüche 1 bis 3,
**dadurch gekennzeichnet,**
**dass** ein aromatisches oder aliphatischen Lösungsmittel oder eine Mischung davon verwendet wird.

5. Verfahren nach einem der Ansprüche 1 bis 4,
**dadurch gekennzeichnet,**
**dass** das Katalysatorsystem eine Konzentration in der Reaktionsmischung von 0.01 bis 40 mmol/l an Titan aufweist.

6. Verfahren nach einem der Ansprüche 1 bis 5,
**dadurch gekennzeichnet,**
**dass** das Katalysatorsystem eine Konzentration in der Reaktionsmischung von 0.05 bis 10 mmol/l an Titan aufweist.

7. Verfahren nach einem der Ansprüche 1 bis 6,
**dadurch gekennzeichnet**,
zunächst das Lösungsmittel inkl. polarer Komponente, danach zumindest eine Organometallverbindung, danach zumindest eine Titan-enthaltende Verbindung, danach Ammoniak und danach Isopren zugegeben wird.

8. Verfahren nach einem der Ansprüche 1 bis 7,
**dadurch gekennzeichnet,**
**dass** zunächst das Lösungsmittel inkl. polarer Komponente, danach zumindest eine Titan enthaltende Verbindung, danach zumindest eine Organometallverbindung, danach Ammoniak und danach Isopren zugegeben wird.

9. Verfahren nach einem der Ansprüche 1 bis 8,
**dadurch gekennzeichnet,**
**dass** es sich bei der Organometallverbindung um eine Organoaluminiumverbindung handelt.

10. Verfahren nach Anspruch 9,
**dadurch gekennzeichnet,**
**dass** es sich bei der Organometallverbindung um Ethoxydiethylaluminium oder Ethylaluminiumsesquichlorid handelt.

11. Verfahren nach einem der Ansprüche 9 bis 10,
**dadurch gekennzeichnet,**
**dass** das molare Verhältnis zwischen Titan und Aluminium von 1:10 bis 1:60 beträgt.

12. Verfahren nach einem der Ansprüche 1 bis 11,
**dadurch gekennzeichnet,**
**dass** 1,5 und/oder 1,6 Dimethylcycloocta-1,5-dien (DMCOD) isoliert werden.

## Claims

1. Continuous and/or batchwise process for preparing trimethylcyclododecatriene, by reacting isoprene in the presence of a solvent, of at least one catalyst system and of at least one organometallic compound to give crude trimethylcyclododecatriene, **characterized in that**
- the catalyst system comprises titanium,
- ammonia is added to the catalyst system,
- before the addition of the catalyst component, the solvent contains 10-1000 ppm of a polar component of the general formula HO-R where R is selected from the group which consists of branched and unbranched C₁-C₁₈-alkyl, C₁-C₁₈-_{C}y_{C}loalkyl, C₁-C₁₈-aryl, C₁-C₁₈-aralkyl and H, and
- the reaction temperature is less than or equal to 80°C.

2. Process according to Claim 1,
**characterized in that**
the catalyst system comprises titanium tetrachloride.

3. Process according to one of Claims 1 to 2,
**characterized in that**
the reaction is carried out at from 30 to 70°C.

4. Process according to one of Claims 1 to 3,
**characterized in that**
an aromatic or aliphatic solvent or a mixture thereof is used.

5. Process according to one of Claims 1 to 4,
**characterized in that**
the catalyst system has a concentration in the reaction mixture of from 0.01 to 40 mmol of titanium/l.

6. Process according to one of Claims 1 to 5,
**characterized in that**
the catalyst system has a concentration in the reaction mixture of from 0.05 to 10 mmol of titanium/l.

7. Process according to one of Claims 1 to 6,
**characterized in that**
first the solvent including polar component, then at least one organometallic compound, then at least one titanium-containing compound, then ammonia and then isoprene is added.

8. Process according to one of Claims 1 to 7,
**characterized in that**
first the solvent including polar component, then at least one titanium-containing compound, then at least one organometallic compound, then ammonia and then isoprene is added.

9. Process according to one of Claims 1 to 8,
**characterized in that**
the organometallic compound is an organoaluminum compound.

10. Process according to Claim 9,
**characterized in that**
the organometallic compound is ethoxydiethylaluminum or ethylaluminum sesquichloride.

11. Process according to one of Claims 9 to 10,
**characterized in that**
the molar ratio between titanium and aluminum is from 1:10 to 1:60.

12. Process according to one of Claims 1 to 11,
**characterized in that**
1,5 and/or 1,6-dimethylcycloocta-1,5-diene (DMCOD) is isolated.

## Revendications

1. Procédé continu et/ou discontinu pour la fabrication de triméthylcyclododécatriène par mise en réaction d'isoprène en présence d'un solvant, d'au moins un système catalytique et d'au moins un composé organométallique pour former un triméthylcyclododécatriène brut, **caractérisé en ce que**
- le système catalytique contient du titane,
- de l'ammoniac est ajouté au système catalytique,
- le solvant contient avant l'ajout du composant catalytique 10 à 1 000 ppm d'un composant polaire de formule générale HO-R, R étant choisi dans le groupe constitué par alkyle en C₁-C₁₈ ramifié et non ramifié, cycloalkyle en C₁-C₁₈, aryle en C₁-C₁₈, aralkyle en C₁-C₁₈ et H, et
- la température de réaction est inférieure ou égale à 80 °C.

2. Procédé selon la revendication 1, **caractérisé en ce que** le système catalytique contient du tétrachlorure de titane.

3. Procédé selon l'une quelconque des revendications 1 à 2, **caractérisé en ce que** la réaction est réalisée à 30 à 70 °C.

4. Procédé selon l'une quelconque des revendications 1 à 3, **caractérisé en ce qu'**un solvant aromatique ou aliphatique ou un mélange de ceux-ci est utilisé.

5. Procédé selon l'une quelconque des revendications 1 à 4, **caractérisé en ce que** le système catalytique présente une concentration dans le mélange réactionnel de 0,01 à 40 mmol/l de titane.

6. Procédé selon l'une quelconque des revendications 1 à 5, **caractérisé en ce que** le système catalytique présente une concentration dans le mélange réactionnel de 0,05 à 10 mmol/l de titane.

7. Procédé selon l'une quelconque des revendications 1 à 6, **caractérisé en ce que** le solvant comprenant un composant polaire est tout d'abord ajouté, puis au moins un composé organométallique, puis au moins un composé contenant du titane, puis de l'ammoniac, puis de l'isoprène.

8. Procédé selon l'une quelconque des revendications 1 à 7, **caractérisé en ce que** le solvant comprenant un composant polaire est tout d'abord ajouté, puis au moins un composé contenant du titane, puis au moins un composé organométallique, puis de l'ammoniac, puis de l'isoprène.

9. Procédé selon l'une quelconque des revendications 1 à 8, **caractérisé en ce que** le composé organométallique est un composé d'organoaluminium.

10. Procédé selon la revendication 9, **caractérisé en ce que** le composé organométallique est l'éthoxydiéthylaluminium ou le sesquichlorure d'éthylaluminium.

11. Procédé selon l'une quelconque des revendications 9 à 10, **caractérisé en ce que** le rapport molaire entre le titane et l'aluminium est de 1:10 à 1:60.

12. Procédé selon l'une quelconque des revendications 1 à 11, **caractérisé en ce que** le 1,5-et/ou le 1,6-diméthylcycloocta-1,5 -diène (DMCOD) sont isolés.
